# EUROPEAN PATENT APPLICATION

(11) **EP 2 113 190 A1**
(43) Date of publication of application: **04.11.2009**
(21) Application number: 09251211.0
(22) Date of filing: 28.04.2009
(51) Int. Cl.: A61B 1/313, A61B 1/12

(54) **Methods and devices for maintaining visibility during surgical procedures**

(30) Priority: 29.04.2008 US 111425
(71) Applicant: Ethicon Endo-Surgery, Inc., Cincinnati, OH 45242 (US)
(72) Inventor: Duke, Daniel H., West Chester, OH 45069 (US); Bookbinder, Mark J., Blue Ash, OH 45252 (US); Tanguay, Randall, Lebanon, OH 45036 (US); Farison, Brian K., Milford, OH 45150 (US); Mumaw, Daniel J., Cincinnati, OH 45204 (US); Moreno, Cesar E., Cincinnati, OH 45214 (US); Franer, Paul T., Cincinnati, OH 45233 (US); Gilker, Thomas A., Cincinnati, OH 45211 (US)
(74) Representative: Tunstall, Christopher Stephen

(57) **Abstract**

Methods and devices are provided for maintaining visibility during surgical procedures. In general, the methods and devices can allow for a surgical instrument to maintain visibility during a surgical procedure using a protective element coupled to the surgical instrument. The protective element can protect a viewing element on the surgical instrument while the surgical instrument is being passed through an introducer device into or out of a body cavity (e.g., during insertion and/or withdrawal). The protective element can also optionally protect the surgical instrument's viewing element while at least a portion of the surgical instrument is disposed in a body cavity.

## Description

### FIELD OF THE INVENTION

The present invention relates to methods and devices for performing surgical procedures, and in particular to methods and devices for maintaining visibility during surgical procedures.

### BACKGROUND OF THE INVENTION

During laparoscopic surgery, one or more small incisions are formed in the abdomen and a trocar is inserted through the incision to form a pathway that provides access to the abdominal cavity. The trocar is used to introduce various instruments and tools into the abdominal cavity, as well as to provide insufflation to elevate the abdominal wall above the organs. During such procedures, a scoping device, such as an endoscope or laparoscope, is inserted through one of the trocars to allow a surgeon to view the operative field on an external monitor coupled to the scoping device.

Scoping devices are often inserted and removed through a trocar multiple times during a single surgical procedure, and during each insertion and each removal they can encounter fluid that can adhere to the scope's lens and fully or partially impede visibility through the lens. Furthermore, a scope can draw fluid from inside or outside a patient's body into the trocar, where the fluid can be deposited within the trocar until the scope or other instrument is reinserted through the trocar. Upon reinsertion, fluid can adhere to the scope's lens. The scope's lens thus needs to be cleaned to restore visibility, often multiple times during a single surgical procedure. With limited access to a scope in a body, each lens cleaning can require removing the scope from the body, cleaning the scope lens of fluid, and reintroducing the scope into the body. Such lens cleaning is a time-consuming procedure that also increases the chances of complications and contamination through repeated scope insertion and removal.

Accordingly, there is a need for methods and devices for maintaining clear visibility through a lens of a scoping device during a surgical procedure.

### SUMMARY OF THE INVENTION

The present invention generally provides methods and devices for maintaining visibility during a surgical procedure using a protective element coupled to the surgical instrument. In one embodiment, a surgical device is provided that includes a surgical instrument that can pass through a working channel of an introducer device extending into a body cavity. A protective element can be coupled to the surgical instrument that can prevent fluid from contacting a distal end of the surgical instrument while the surgical instrument is being passed through the introducer device. The surgical instrument can vary, and can include, for example, a scope with a viewing element at a distal end of the scope. In some embodiments, the protective element can prevent fluid from contacting the distal end of the surgical instrument while the distal end of the surgical instrument is disposed in the body cavity.

The protective element can have a variety of configurations. For example, the protective element can be removable from the surgical instrument. In some embodiments, the protective element can include an absorbent material disposed on an outside surface of the protective element for absorbing fluid. As another example, the protective element can include a tear-away sheath disposed over at least a distal portion of the surgical instrument. In yet another example, the protective element can include movable jaws coupled to the distal end of the surgical instrument that can move between a closed position in which the jaws enclose the distal end of the surgical instrument and an open position in which the distal end of the surgical instrument is exposed. In another embodiment, the protective element can include an extension that can push open a seal within the introducer device to prevent the seal from contacting a lens on the distal end of the surgical instrument. As still another example of the protective element, the protective element can be disposed on the distal end of the surgical instrument, and it can include a plurality of movable protective members. The protective members can also have a variety of configurations, such as a plurality of lenses stacked on top of one another. In some embodiments, each lens can be hingedly coupled to a support mated to the distal end of the surgical instrument.

In another embodiment, a surgical device can include a scoping device having a viewing element at a distal end and at least two optically clear protective elements coupled to the distal end of the scoping device that can provide a barrier to protect the viewing element from fluid in an external environment. The optically clear protective elements can be sequentially movable. The optically clear protective elements can have a variety of configurations. For example, the optically clear protective elements can each be in the form of a tear-away sheath. The tear-away sheaths can be disposed in layers over the viewing element of the scoping device. For another example, the optically clear protective elements can be disposed within a housing coupled to the scoping device, e.g., hingedly coupled to the housing. In some embodiments, each optically clear protective element can be movable between a closed position in which the optically clear protective element is within a viewing path of the viewing element and an open position in which the optically clear protective element is out of the viewing path of the viewing element. The optically clear protective elements can be biased to the open position and held in the closed position by a lip. In some embodiments, the surgical instrument can be movable relative to the housing to sequentially move the optically clear protective elements to the open position.

In yet another embodiment, a surgical device can include a scoping device having a viewing element at a distal end and being disposable in a body through a working channel of an introducer device. A protective element can be coupled to the scoping device and can be movable between a first configuration in which the protective element provides a fluid seal around the viewing element while the distal end of the scoping device is disposed through a working channel of an introducer device, and a second configuration in which the viewing element is exposed to a fluid environment surrounding the distal end of the scoping device. The protective element can have a variety of configurations, such as an end cap coupled to the distal end of the scoping device that has at least two jaws that are closed in the first configuration and open in the second configuration. In other embodiments, the protective element can be a tear-away sheath that is removed from the scoping device in the second configuration. The tear-away sheath can optionally include an outer absorbent layer.

In still another embodiment, a surgical device can include a scoping device having a viewing element at a distal end and being disposable in a body through a working channel of an introducer device. A diverting mechanism can be located on the distal end of the scoping device, and it can be effective to open a seal in an introducer device to prevent the viewing element from contacting the seal. The diverting mechanism can have a variety of sizes and shapes, but in some embodiments the diverting mechanism can extend a distance beyond the distal end of the scoping device, e.g. by about 0.25 inches. The diverting mechanism can also have a variety of configurations, and it can be integrally formed with or removably coupled to the scoping device. In some embodiments, the diverting mechanism can include an elongate tubular body coupled to the distal end of the scoping device to form an extension that extends distally beyond the distal end of the scoping device.

In other aspects, a surgical method is provided. The method includes passing a surgical instrument through an introducer device having a working channel extending into a body cavity. A distal end of the surgical instrument includes a protective element that can prevent fluid from contacting the distal end of the surgical instrument while the surgical instrument is being passed through the introducer device. In some embodiments, the method further includes moving the protective element to expose a second protective element located on the distal end of the surgical instrument when the surgical instrument is at least partially disposed in a body cavity. The method can also optionally include removing the protective element from the surgical instrument when the surgical instrument is at least partially disposed in a body cavity.

The protective element can prevent fluid from contacting the surgical instrument's distal end in a variety of ways. For example, the protective element can prevents fluid from contacting the distal end of the surgical instrument while the distal end of the surgical instrument is disposed in a body cavity. As another example, the protective element can open a seal within the introducer device to prevent a viewing element at the distal end of the surgical instrument from contacting the seal. In some embodiments, preventing fluid from contacting a distal end of the surgical instrument can include moving jaws disposed over the distal end of the surgical device from a closed position in which the jaws enclose the distal end of the surgical instrument to an open position in which the distal end of the surgical instrument is exposed.

In still another embodiment, a surgical device can include a trocar having a working channel configured to extend into a body cavity and to allow passage of a surgical instrument therethrough, and a seal assembly disposed in the trocar having a seal with an opening configured to form a seal around a surgical instrument passed through the trocar, and a protective element including at least one camming rib formed thereon and configured to contact the surgical instrument prior to the surgical instrument contacting the seal. The camming rib can be effective to prevent fluid in the opening from being deposited on a distal surface of the surgical instrument. The camming rib can be effective to provide point contact with the surgical instrument.

### BRIEF DESCRIPTION OF THE DRAWINGS

The invention will be more fully understood from the following detailed description taken in conjunction with the accompanying drawings, in which:

FIG. 1 is a partial cross-sectional view of one embodiment of a surgical instrument having a plurality of lenses coupled thereto;

FIG. 2 is a partial cross-sectional view of the distal end of the instrument of FIG. 1;

FIG. 3 is a perspective partially transparent view of the distal end of the instrument of FIG. 1;

FIG. 4 is a perspective view of the surgical instrument of FIG. 1 disposed through an introducer device;

FIG. 5 is a partial cross-sectional view of one embodiment of a surgical instrument having a sheath disposed therearound;

FIG. 6 is a perspective view of another embodiment of a surgical instrument having a sheath disposed therearound with a weakened region;

FIG. 7 is a perspective view of yet another embodiment of a surgical instrument having a plurality of sheaths disposed therearound;

FIG. 8 is a perspective view of one embodiment of a surgical instrument disposed in a sheath and through an introducer device;

FIG. 9 is a perspective view of the sheath being removed from the surgical instrument of FIG. 8;

FIG. 10 is a side view of one embodiment of a surgical instrument having an extension element at its distal end;

FIG. 11 is a side view of another embodiment of a surgical instrument including an extension element at its distal end;

FIG. 12 is a perspective view of one embodiment of a surgical instrument and an extension element that can be coupled to the surgical instrument's distal end;

FIG. 13 is a perspective view of the surgical instrument of FIG. 10 being advanced into a trocar;

FIG. 14 is a partial cross-sectional view of the surgical instrument further advanced into the trocar of FIG. 13;

FIG. 15 is a partial cross-sectional view of the surgical instrument advanced through a first seal assembly included in the trocar of FIG. 14;

FIG. 16 is a partial cross-sectional view of a portion of the surgical instrument and trocar of FIG. 15 showing the surgical instrument advanced toward a second seal assembly in the trocar of FIG. 15;

FIG. 17 is a partial cross-sectional view of the surgical instrument advancing through the second seal assembly in the trocar of FIG. 16;

FIG. 18 is a partial cross-sectional view of the surgical instrument advanced through the second seal assembly in the trocar of FIG. 17;

FIG. 19 is a perspective view of one layer of a multi-layer protective element having camming ribs formed thereon;

FIG. 20 is a top view of a multi-layer protective seal having off-set camming ribs;

FIG. 21 is a side partially cross-sectional view of one embodiment of movable jaws coupled to a distal end of a surgical instrument, showing the jaws in an open position;

FIG. 22 is a schematic view of the movable jaws of FIG. 21 coupled to the distal end of the surgical instrument, showing the jaws in a closed position;

FIG. 23 is a partial cross-sectional view of one embodiment of a hinge assembly of the movable jaws of FIG. 21;

FIG. 24 is a partial cross-sectional view of another embodiment of a hinge assembly of the movable jaws of FIG. 21;

FIG. 25 is a partial cross-sectional view of yet another embodiment of a hinge assembly of the movable jaws of FIG. 21;

FIG. 26 is a partial cross-sectional view of a still another embodiment of a hinge assembly of the movable jaws of FIG. 21;

FIG. 27 is a side partially cross-sectional view of another embodiment of movable jaws that can be coupled to a distal end of a surgical instrument;

FIG. 28 is a side view of the movable jaws and the surgical instrument of FIG. 27 being advanced into a trocar;

FIG. 29 is a side partially cross-sectional view of the movable jaws and the surgical instrument further advanced into the trocar of FIG. 28;

FIG. 30 is a partially cross-sectional view of the movable jaws and the surgical instrument advanced through a first seal assembly of the trocar of FIG. 29; and

FIG. 31 is a side view of the movable jaws and the surgical instrument advanced into a body cavity through the trocar of FIG. 30.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Various exemplary methods and devices are provided for maintaining visibility during surgical procedures. In an exemplary embodiment, the methods and devices can allow for a surgical instrument to maintain visibility during a surgical procedure using a protective element coupled to the surgical instrument. The protective element can protect a viewing element on the surgical instrument while the surgical instrument is being passed through an introducer device into or out of a body cavity (e.g., during insertion and/or withdrawal). The protective element can also optionally protect the surgical instrument's viewing element while at least a portion of the surgical instrument is disposed in a body cavity. The term "viewing element" as used herein is intended to encompass any one or more elements (e.g., a lens, a sensor, etc.) configured to allow for any type of visualization through still, moving, or other visual images. The term "body cavity" as used herein is intended to encompass any internal body area, e.g., the abdominal cavity, the oral cavity, a body lumen, etc. Using the protective element can help prevent fluid in the introducer device and/or in a patient's body from contacting the surgical instrument's viewing element, thereby helping the viewing element maintain visual acuity. In this way, a surgical procedure is less likely to be interrupted one or more times to address a visually impeded viewing element. Because the protective element can protect the viewing element, the surgical instrument is less likely to require withdrawal from the body for replacement with another, clean surgical instrument and/or for cleaning or replacement of the viewing element. Reducing the need to remove the surgical instrument through the introducer device can also reduce the chances of the surgical instrument drawing fluid into the introducer device during withdrawal that could obscure the viewing element's viewing path during withdrawal and/or upon the surgical instrument's reinsertion (or other surgical instrument's insertion) into the introducer device. Furthermore, the protective element in some embodiments can be replaced while disposed in a body cavity should the protective element become damaged, smudged, or otherwise visually impeded, which can also save time during a surgical procedure because the surgical instrument need not be withdrawn, cleaned, and reinserted (or exchanged for another surgical instrument) to restore visual clarity through the viewing element.

A person skilled in the art will appreciate that the term "fluid" as used herein is intended to include any substance that, when on a surgical instrument, can adversely affect the functioning of the instrument or a surgeon's ability to use it. Fluids include any kind of bodily fluid, such as blood, and any kind of fluid introduced during a surgical procedure, such as saline. Fluids also include fluid/solid mixtures or fluids with particles (such as pieces of tissue) suspended or located therein, as well as viscous materials and gases.

A person skilled in the art will appreciate that while the methods and devices are described in connection with endoscopic procedures, the methods and devices disclosed herein can be used in numerous surgical procedures and with numerous surgical instruments. By way of non-limiting example, the devices can be used in laparoscopic procedures, in which the device is introduced percutaneously. The methods and devices can also be used in open surgical procedures. Furthermore, a person skilled in the art will appreciate that the methods and devices disclosed herein can be used with numerous rigid and/or flexible surgical viewing instruments. By way of non-limiting example, the surgical viewing instrument can be a laparoscope, a colonoscope, an arthroscope, and any other type of surgical device that has a viewing element.

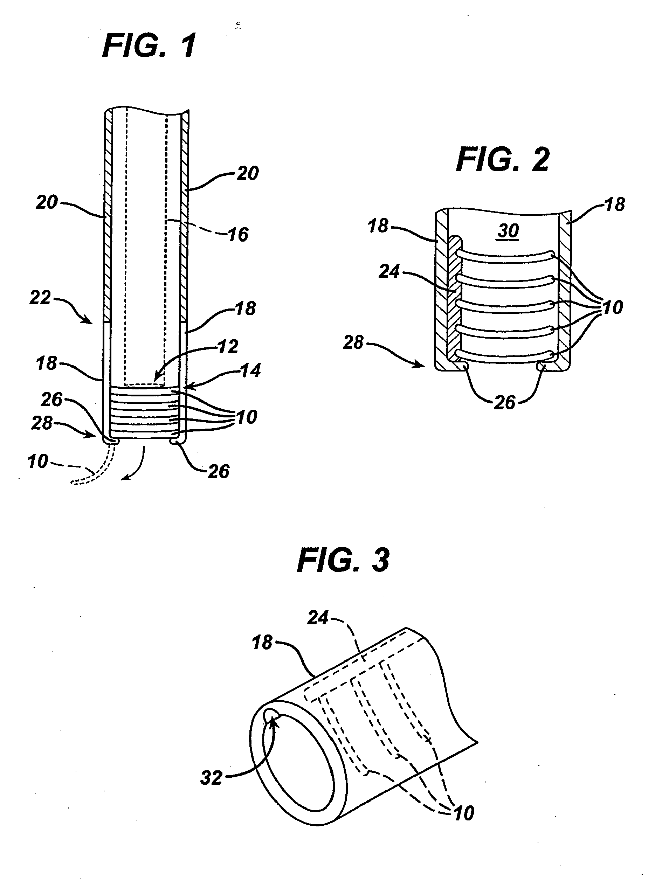

FIGS. 1-4 illustrate one exemplary embodiment of a protective element coupled to a surgical viewing instrument. Generally, as shown in FIG. 1, one or more lenses 10 can be disposed at a distal end 14 of a surgical viewing instrument, e.g., an endoscope 16, to protect a viewing element 12 at the endoscope's distal end 14. In an exemplary embodiment at least one of the lenses 10 (e.g., a distal-most one of the lenses 10) can be disposed between the viewing element 12 and an external environment such that at least one of the lenses 10 is within the viewing element's viewing path into the external environment. In this way, at least one of the lenses 10 can protect the endoscope's viewing element 12 from the external environment. If the lens 10 exposed to the external environment becomes visually impeded by fluid in the external environment or becomes otherwise unusable or undesirable, that lens 10 can be moved or removed to allow another one of the lenses 10 to be exposed to the external environment and provide a barrier between the endoscope's viewing element 12 and the external environment. Each of the lenses 10 can be sequentially movable, thereby allowing for multiple lens changes due to fluid obstruction or other interference. Furthermore, the lenses 10 can be moved while the endoscope 16 is disposed in a body cavity, thereby reducing the need to remove the endoscope 16 from a body during a surgical procedure to clear the viewing element's viewing path and reducing the chances of the endoscope 16 drawing fluid from the external environment into an introducer device used to withdraw the endoscope 16 from the body and to reintroduce the endoscope 16 (or other surgical instrument) into the body.

The lenses 10 can be formed from a variety of materials but are preferably formed from an optically clear biocompatible material. In an exemplary embodiment, the lenses 10 are each formed from an optically clear pliable material, e.g., a polymer, having a magnifying power of one, e.g., non-magnifying 1x, so as to be substantially non-modifying of the view through the viewing element 12, but the lenses 10 can be formed from any type and any combination of rigid and pliable materials having any magnifying power. The lenses 10 are preferably non-tinted, but any one or more of the lenses 10 can have any color tinting. A lens 10 can have a hydrophobic coating, an ultrahydrophobic coating, or other type of fluid-repellant coating covering at least a portion of its surface to help prevent fluids from contacting the viewing element 12.

The lenses 10 can also have various sizes, but preferably the lenses 10 have a size that is equal to or greater than the endoscope's viewing element 12 to help prevent the lenses 10 from obscuring the viewing path of the viewing element 12. In other words, the lenses 10 are preferably invisible to the viewing element 12, notwithstanding any color tinting and any controlled movement of the lenses 10 as discussed further below. The lenses 10 can each have any thickness, which is preferably constant for a given lens 10, although a lens's thickness can vary. The lenses 10 can also have any shape, e.g., circular, elliptical, rectangular, square, etc. In an exemplary embodiment, the lenses 10 are planar, e.g., lacking curvature, to match the scope's typically planar viewing element 12, but the lenses 10 can be convex or concave. The lenses 10 are preferably identical to one another, but one or more of the lenses 10 can vary in any number of ways from one or more of the other lenses 10.

The lenses 10 can also have any configuration. In an exemplary embodiment, as shown in FIGS. 1-3, the lenses 10 can be stacked on top of one another. Any number of lenses 10 can be included in a stack, e.g., two, ten, etc. A different number of lenses 10 can be included in a stack for different surgical instruments. The lenses 10 can be aligned in substantially parallel planes, which can also each be substantially parallel with a plane of the endoscope's distal end 14 and the viewing element 12. The lenses 10 can be stacked to touch adjacent lenses 10, or any amount of space can exist between adjacent lenses 10.

The lenses 10 can be stacked in any way, but in one exemplary embodiment they are stacked within a housing 18 coupled to the endoscope 16. The housing 18 can be formed from any type and any combination of preferably biocompatible materials. The housing 18 can be rigid or flexible, but the housing 18 is preferably rigid to keep the lenses 10 in alignment with the endoscope's distal end 14 and the viewing element's viewing path. The housing 18 can also have any size and shape, but the housing 18 is preferably cylindrical to match the typically cylindrical shape of the distal end 14 of the endoscope 16. The housing 18 can extend around any portion of the endoscope's longitudinal length, as discussed below.

In use, the housing 18 can be detachably or fixedly coupled to the endoscope 16, preferably at the endoscope's distal end 14. If the housing 18 is detachably coupled to the endoscope 16, the housing 18 can be detached from the endoscope 16 and optionally replaced with another, similar housing. Such housing replacement can be advantageous, for example, if the housing 18 becomes damaged or if all the lenses 10 within the housing 18 have been smudged by fluid or otherwise become unusable or undesirable. Detachably coupling the housing 18 to the endoscope 16 can allow the housing 18 to be retrofitted to existing endoscopes and/or to be detached from the endoscope 16 and be coupled to one or more other surgical instruments (preferably after sterilizing the housing 18).

Various techniques can be used to mate the housing 18 to the endoscope 16. For example, the housing 18 can optionally be detachedly or fixedly coupled to a sheath or outer sleeve 20. The outer sleeve 20 can couple to the housing 18 at the housing's proximal end 22 and extend around the endoscope 16 to the endoscope's proximal end or around any portion thereof, such as to a location proximally beyond an introducer device used to introduce the endoscope 16 into a body such that the outer sleeve 20 can be manipulated from outside the body. As discussed further below, the outer sleeve 20 can be used to help hold the lenses 10 and the endoscope 16 in position relative to each other until (and if) a lens 10 is moved. In other embodiments, the housing 18 and the outer sleeve 20 can be integrally formed as a single elongate housing or sleeve that is slidably disposed over the endoscope 16.

The lenses 10 can be arranged in a stacked configuration within the housing 18 in a variety of ways, but in an exemplary embodiment, each lens 10 can be hingedly coupled to the housing 18. For example, as shown in FIG. 2, the hinge 24 can be in the form of a rigid and/or pliable rod that is disposed on and extends along an inner surface of the housing 18. Alternatively, the hinge 24 can be integrally formed with the housing 18, for example in a sidewall of the housing 18. The hinge 24 can have any size, shape, and configuration, but in an exemplary embodiment the hinge 24 is a molded or mechanical hinge that can allow the lenses 10 to move as discussed further below. The hinge 24 can extend for any length, and in some embodiments can extend beyond the housing's proximal end 22 and, as discussed further below, can be used to help hold the lenses 10 and the endoscope 16 in position relative to each other until (and if) a lens 10 is moved. The hinge 24 with the lenses 10 attached thereto can optionally be removable to allow it to be replaced with a new plurality of lenses such as by removing the hinge 24 with the lenses 10 attached and disposing a new hinge with new lenses attached within the housing 18. The lenses 10 can be pre-loaded into the housing 18, or the lenses 10 can be disposed in the housing 18 following the housing's coupling to the endoscope 16.

In use, the hinge 24 can allow each lens to move between a closed position, in which the lens is within the viewing path of the viewing element, and a second, open position in which the lens is moved out of the viewing path. The hinge can allow such movement. In an exemplary embodiment, the lenses 10 can be biased to an open position such that the lenses 10 can be positioned outside the viewing element's viewing path, and a lip 26 at a distal end 28 of the housing 18 can help hold the lenses 10 in a tensioned stack within the housing 18 (e.g., as shown in FIGS. 1-3) where each of the lenses 10 is in the closed position and within the viewing element's viewing path but, as mentioned above, is optically clear and invisible to the viewing element 12 (except for any purposeful visual interference such as tinting and/or magnifying). The lip 26 can be preferably integrally formed with the housing 18 or otherwise coupled to the housing 18. The lip 26 preferably extends circumferentially around the housing's distal end 28, but the lip 26 can alternatively be in the form of one or more protrusions extending into the housing's inner pathway 30 and configured to help hold the lenses 10 in a stacked configuration within the housing 18. Pushing a lens 10 beyond the housing's lip 26 can allow the hinge 24 to open and the lens 10 to "pop" free of the housing 18 and return to the unbiased open position. As shown in FIG. 3, the lip 26 can include a recess 32 in its circumference that can be generally aligned with the hinge 24 to accommodate distal movement of the hinge 24 as discussed further below.

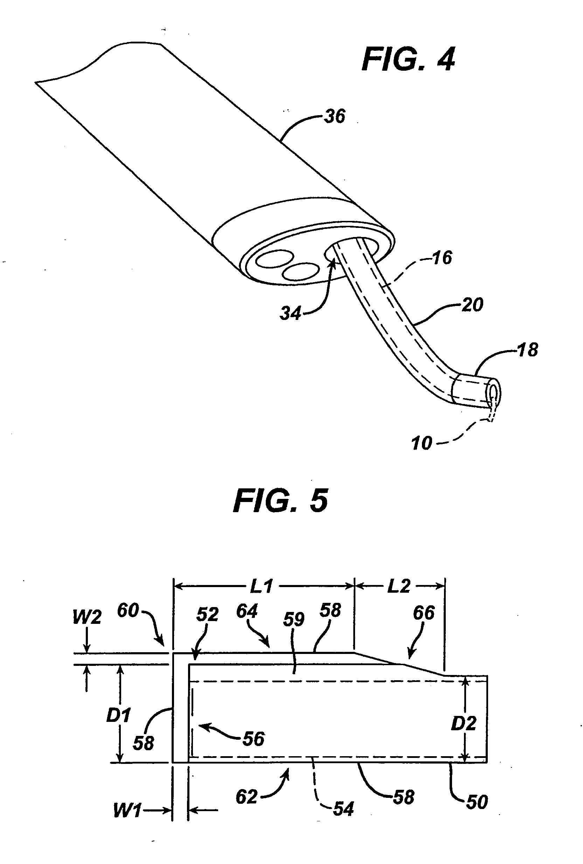

In use, as shown in FIG. 4, the endoscope 16, disposed within the outer sleeve 20 and the housing 18, can be advanced into a body cavity through a working channel 34 of an introducer device 36. The introducer device 36 can include any surgical instrument having a cannulated pathway, e.g., a trocar that provides a pathway through tissue to a body cavity. Alternatively, the endoscope 16, disposed within the outer sleeve 20 and the housing 18, can be directly inserted into a body cavity through a natural orifice, through a man-made orifice such as a puncture hole formed in tissue, and in any other way appreciated by a person skilled in the art. The endoscope 16 can be coupled to the housing 18 and the outer sleeve 20 (if present) such that the endoscope 16, the housing 18, and the outer sleeve 20 can move as a single unit. In other words, advancing the endoscope 16 into a desired position within a body cavity can also advance the housing 18 and the outer sleeve 20 so as to keep the stacked lenses 10 within the housing 18 protective of the viewing element 12 and within the viewing element's viewing path.

If visual clarity through the viewing element 12 decreases, at least one of the lenses 10 exposed to an external environment (e.g., the distal-most lens on the stack of lenses) may have become obscured by fluid or otherwise become unusable or undesirable. As mentioned above, the obscured lenses 10 can be moved to help restore visual clarity through the viewing element 12. The lens 10 can be moved in a variety of ways. For example, the endoscope 16 can be advanced distally relative to the outer sleeve 20 to push on the stack of lenses 10 and/or on the hinge 24 coupled to the lenses 10, thereby pushing the obscured lens 10 beyond the lip 26 and out of the housing 18 such that the lens 10 moves from the closed position to the open position. Alternatively, or in addition, if the hinge 24 extends beyond a proximal end of the introducer device 36 (e.g., outside the body), the hinge 24 can be manipulated (e.g., pushed distally relative to the sleeve 20 and the housing 18) to push on the stack of lenses 10 to move the lens 10 beyond the lip 26. In some embodiments, an arm or other triggering mechanism can be coupled to the hinge 24 and can extend through the introducer device's working channel 34 out of the body where the arm or triggering mechanism can be manipulated to distally advance or otherwise eject the obscured lens 10 from the housing 18. A person skilled in the art will appreciate that lenses 10 can be advanced distally from the housing 18 in any of these or other ways.

However moved, the lens 10 to be moved can be advanced distally beyond the housing's lip 26 and "pop" out of the housing 18 through the housing's recess 32 (if necessary and if present) and thus also out of the viewing element's viewing path. FIGS. 1 and 4 illustrate a lens 10 in shadow "popped" out of the housing 18 such that the "popped" lens 10 is in the open position, pivoted to one side of the housing 18 while remaining coupled to the housing 18, e.g., via the hinge 24. Preferably, the endoscope 16 is advanced a distance to allow only one of the lenses 10 to be ejected out of the housing 18 at a time (e.g., the distal-most one of the lenses 10 disposed within the housing 18), but more than one of the lenses 10 can be so moved at a time. Distally advancing the endoscope 16 to move one of the lenses 10 from the viewing element's viewing path can also advance at least a portion of the hinge 24 beyond the housing's distal end 28 through the housing's recess 32. When the lens 10 to be moved is ejected from the housing 18, it can remain coupled to the hinge 24 (and therefore also to the housing 18), thereby preventing the lens 10 from being disposed in or getting lost inside the body cavity and allowing the moved lens to be removed from the body cavity when the housing 18 is removed from the body cavity. However, in other embodiments, the lens need not be attached to a hinge and can simply be ejected from the device and into the body. In such an embodiment, the lens is preferably biodegradable.

As a surgical procedure continues, each time visual clarity through the viewing element 12 decreases (if ever), another one of the lenses 10 can be moved as discussed above to help restore visual clarity.

The lenses 10 can be replaced with a plurality of new lenses at any time (before, during, or following a surgical procedure) for any reason, such as if all of the lenses 10 have been moved out of the housing 18, thereby exposing the viewing element 12 to the external environment. Such exposure of the viewing element 12 can be detected in a variety of ways. For example, the proximal-most one of the lenses 10 can be tinted such that moving that proximal-most lens 10 when it is in the distal-most position can remove the tinting previously apparent through the viewing element 12. As another example, attempting to move one of the lenses 10 may not restore visual clarity, indicating that the viewing element 12 itself may be exposed to an external environment and obscured by fluid.

The lenses 10 can be replaced in a variety of ways. As mentioned above, the hinge 24 with the lenses 10 coupled thereto can be removed from the housing 18 and replaced with another hinge having another plurality of lenses attached thereto. Alternatively, the housing 18 can be decoupled from the sleeve 20 and/or the endoscope 16, and another housing with another plurality of lenses can be coupled to the endoscope 16.

As mentioned above, when the endoscope 16 is withdrawn from a body cavity and into the introducer device 36, e.g., to replace the lenses 10, to clean the viewing element 12, to end the surgical procedure, etc., any lenses 10 that have been ejected from the housing 18 can also be withdrawn into the introducer device 36. Whether the lenses 10 are pliable or rigid, any ejected lens or lenses 10 can be naturally withdrawn into the housing 18 and/or the working channel 34 when the endoscope 16 is withdrawn into the introducer device 36. Alternatively or in addition, prior to withdrawing the distal end 28 of the housing 18 into the working channel 34 of the introducer device 36, the hinge 24 can be pulled in a proximal direction to pull any ejected lenses 10 into the housing 18, e.g., by pulling on a portion of the hinge 24 that extends beyond the proximal end of the introducer device outside the body.

In another embodiment, illustrated in FIGS. 5-9, a protective element coupled to a surgical instrument can include a tear-away sheath. Generally, as shown in FIG. 5, a tear-away sheath 50 coupled to a surgical instrument, e.g., an endoscope 54, can protect a viewing element 56 at the endoscope's distal end 52. While the sheath 50 can have a variety of configurations, in an exemplary embodiment the sheath 50 can be configured similar to the lenses 10 discussed above to form a barrier between an external environment and the viewing element 56 of the endoscope 54. The sheath 50 can be disposed around at least the endoscope's distal end 52 such that when the endoscope's distal end 52 is introduced into a body cavity, e.g., advanced through an introducer device, inserted through a natural or man-made orifice, etc., the sheath 50 can protect the viewing element 56 from fluid. In this way, the sheath 50 can protect the viewing element 56 from becoming obscured, damaged, or otherwise adversely affected by fluid during introduction of the endoscope 54 into a body cavity. The sheath 50 can also be configured to be removably coupled with the endoscope 54. In this way, the sheath 50 can be torn away from the endoscope 54 while the endoscope 54 is at least partially disposed within an introducer device and/or in a body cavity, thereby exposing the viewing element 56. Optionally, the sheath 50 can be formed from an optically clear material and can be disposed around at least the endoscope's distal end 52 while the endoscope's distal end 52 is within the body cavity such that the viewing path of the viewing element 56 extends through the sheath 50. In other words, similar to the lenses 10, and as further discussed below, the sheath 50 can be invisible to the viewing element 56 and can be removed if obscured by fluid to help restore visual clarity to the viewing element 56.

The sheath 50 can be formed from a variety of materials but is preferably formed from a fluid impermeable, biocompatible material. In an exemplary embodiment, the sheath 50 is formed from a pliable polymer, e.g., low-density polyethylene (LDPE) and polytetrafluoroethylene. The sheath 50 can be optically clear, translucent, opaque, or any combination thereof. The sheath 50 is preferably optically clear at least at the distal end 52 of the endoscope 54 (e.g., within the viewing path of the viewing element 56). The optically clear portion(s) of the sheath 50 can have any magnifying power and/or any tinting as discussed above for the lenses 10. If optically clear, the sheath 50 is preferably formed from non-magnifying 1x material so as to be substantially non-modifying of the view through the viewing element 56.

While the sheath 50 can have a variety of configurations, in an exemplary embodiment the sheath 50 can be configured to form a barrier between an external environment and the viewing element 56 of the endoscope 54. The sheath 50 can have any shape, but preferably the sheath 50 has an elongate tubular shape having an open proximal end, a closed distal end 60, and an inner pathway 59 extending longitudinally between the proximal and distal ends. The sheath 50 can be disposed around and receive the endoscope 54 within its inner pathway 59 such that the sheath's distal end 60 can cover the endoscope's viewing element 56, and the sheath's proximal end (not shown) can extend out of a proximal end of an introducer device while the endoscope 54 and the sheath 50 are disposed therein. The sheath 50 thus forms a fluid-sealed barrier around at least the distal portion of the endoscope. In use, the sheath 50 can protect the viewing element 56 such that, when (and if) the sheath 50 is torn away from the endoscope 54, the viewing element 56 can have a substantially clear viewing path, having been protected from fluid by the sheath 50.

In some embodiments, the sheath 50 can include an absorbent outer layer on at least a portion of its outer surface 58, preferably on its entire outer surface 58. The absorbent outer layer can be formed from any one or more materials in any combination, preferably pliable, biocompatible materials. The absorbent outer layer can be coupled to the sheath's outer surface 58 in a variety of ways, such as by adhesive bonding, heat sealing, being integrally formed with the sheath 50, or any other way appreciated by a person skilled in the art. The absorbent outer layer can be configured to wipe and clean the inside of an introducer device, e.g., a trocar, a cannulated pathway, etc., when the endoscope 54 is passed through the introducer device, thereby reducing or eliminating any fluid within the introducer device. This will help prevent the fluid from being deposited on the endoscope 54 when the endoscope 54 and/or a sheath is withdrawn through the introducer device and/or reinserted through the introducer device, and/or on another surgical instrument passed through the introducer device. Furthermore, when (and if) the sheath 50 is torn away and removed from the endoscope 54 as discussed further below, fluid can be withdrawn with the sheath 50 as absorbed by the absorbent outer layer, which can also help reduce chances of fluid interfering with the endoscope 54 or other surgical instrument passed through the introducer device.

The size of the sheath 50 can vary, but preferably the sheath 50 has a size and shape that can correspond with the size and shape of the endoscope 54 when the sheath 50 is disposed therearound (with or without stretching or flexing of the sheath 50). The sheath 50 can also have any thickness, e.g., 0.002 in. thick. The sheath 50 can be formed in any way, but by way of example only, a fluid impermeable, biocompatible material can be formed into an elongate tube-like shape by folding a substantially planar piece of the material along a fold line 62 and sealing the sheath's distal end 60 and at least a portion of an unfolded edge 64. The distal end 60 and the edge 64 can be sealed in a variety of ways appreciated by a person skilled in the art, such as by adhesive bonding or heat sealing. The widths of material sealed at the distal end 60 and the edge 64 can vary between embodiments and from each other, but in an exemplary embodiment, a distal end seal width W1 is about 3 mm and an edge seal width W2 is about 2 mm. The distal end 60 is preferably sealed along its entire diameter D1 (e.g., about 16 mm) to help protect the viewing element 56 at the endoscope's distal end 52. Any portion of the edge 64 can be sealed, preferably a portion long enough to allow for complete disposal of the viewing element 56 exposed at the endoscope's distal end 52 within a contained portion of the sheath 50. For example, the edge 64 can be sealed along a length L1 (e.g., about 30 mm) between the sheath's distal end 60 and an S-curve cut transition 66 that tapers for an additional sealed length L2 (e.g., about 15 mm). Proximal to the S-curve cut transition 66, the sheath 50 can have a diameter D2 (e.g., about 15 mm) that is smaller than the sheath's distal diameter D 1, which can help provide a fluid seal around the endoscope 54 at the sheath's distal end 60.

The sheath 50 can be coupled to the endoscope 54 in a variety of ways. In an exemplary embodiment, the endoscope 54 is slidably disposed within the sheath 50. In some embodiments, the sheath 50 can be coupled to the endoscope 54 using an attachment mechanism configured to engage the sheath 50, preferably at the sheath's proximal end, such as a clip, a clamp, adhesive, a groove, a hook, or any other coupling mechanism appreciated by a person skilled in the art. The attachment mechanism can be located on the endoscope 54 and/or on an introducer device used to introduce the endoscope 54 into a body cavity.

As mentioned above, the sheath 50 can be removably mated to the endoscope 54 such that the sheath 50 can be controllably torn away from the endoscope 54. The sheath 50 can be torn away in a variety of ways. For example, a hole can be created in the sheath 50 using the endoscope 54 to allow the endoscope 54 to pass through the sheath 50 as the sheath 50 is pulled away from the endoscope 54 and no longer forms a barrier between the endoscope's viewing element 56 and an external environment. In an exemplary embodiment, the sheath's sealed distal end 60 and/or sealed edge 64 can be detachedly sealed together such that a force applied to the proximal end of the sheath 50 (e.g., pulling the sheath 50 proximally relative to the endoscope 54) can allow the sealed distal end 60 and/or the sealed edge 64 to separate. With the distal end 60 and/or the edge 64 unsealed, the endoscope's distal end 52 can pass through the sheath 50 as the sheath 50 is proximally pulled away from the endoscope 54.

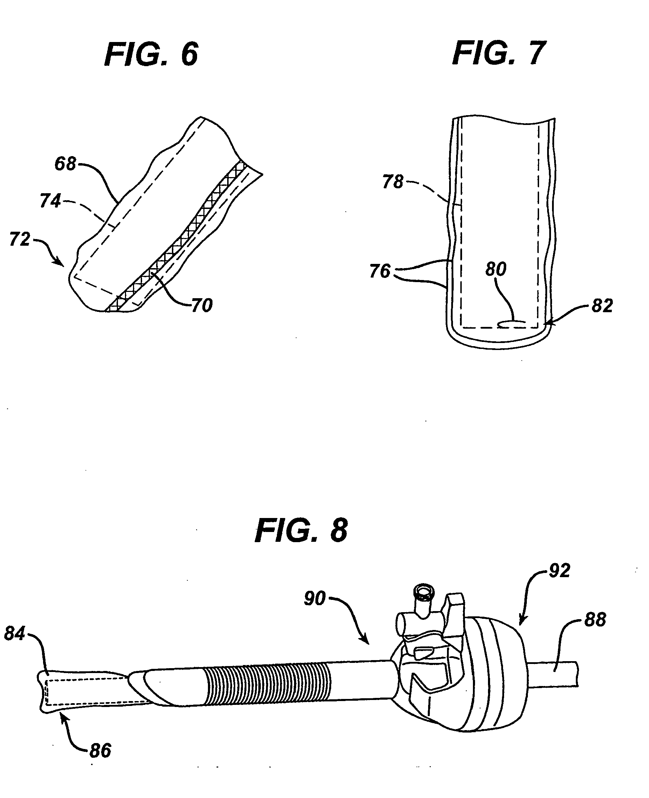

In another embodiment, illustrated in FIG. 6, a sheath 68 can be in the form of a balloon-like elongate tube having one open end (its proximal end) and having a weakened region 70 made from the same or different material as a remaining portion of the sheath 68. Although the weakened region 70 is illustrated as a linear region extending proximally and longitudinally from a distal end 72 of the sheath 68, a person skilled in the art will appreciate that the weakened region 70 can have any shape, size, and configuration at any portion or portions of the sheath 68. However, the weakened region 70 is preferably located over at least a portion of the distal end 72 of the sheath 68 to allow for easier removal of the sheath 68 from around a surgical instrument 74 disposed within the sheath 68. Upon application of a force applied to the sheath 68, the weakened region 70 can tear or otherwise separate, thereby allowing the sheath 68 be torn away and removed from the surgical instrument 74.

A cutting instrument, such as a knife or scissors, can optionally be used to cut at least a portion of a sheath disposed around a surgical instrument. The cutting instrument can be introduced through the endoscope or through the same or different introducer device used to introduce the sheathed surgical instrument into a body cavity. The cutting instrument can cut the sheath from outside the sheath, or alternatively, the cutting instrument can cut the sheath from within, such as by being introduced through a working channel of a scoping device surrounded by the sheath. The sheath is preferably cut at its distal end, although it can be cut at any one or more locations. With an opening through which the scoping device can pass, the sheath can be proximally pulled and removed as discussed above.

Optionally, a plurality of sheaths 76 can be disposed in layers over a surgical instrument 78, as shown in FIG. 7. Although two layered sheaths 76 are illustrated in FIG. 7, any number of sheaths 76 can be layered around the surgical instrument 78. The sheaths 76 can be sequentially torn away from the surgical instrument 78. In this way, when (and if) an outer-most one of the sheaths 76 is torn away, another one of the sheaths 76 can remain disposed around the surgical instrument 78 to protect the endoscope's viewing element 80 (until and if the sheath 76 disposed in direct contact with the surgical instrument 78 is removed). Similar to the lenses 10 discussed above, when one of the sheaths 76 exposed to an external environment becomes obscured by fluid so as to decrease visual clarity through the viewing element 80, that outer-most sheath 76 can be torn away to allow another one of the sheaths 76 to protect the viewing element 80 from the external environment. Sheaths 76 arranged over the endoscope 78 in a layered configuration are preferably optically clear, at least within the viewing element's viewing path, to allow the sheaths 76 to remain disposed around the surgical instrument 78 without substantially reducing or eliminating visibility through the viewing element 80 when at least the surgical instrument's distal end 82 including the viewing element 80 is outside an introducer device and disposed within a body cavity.

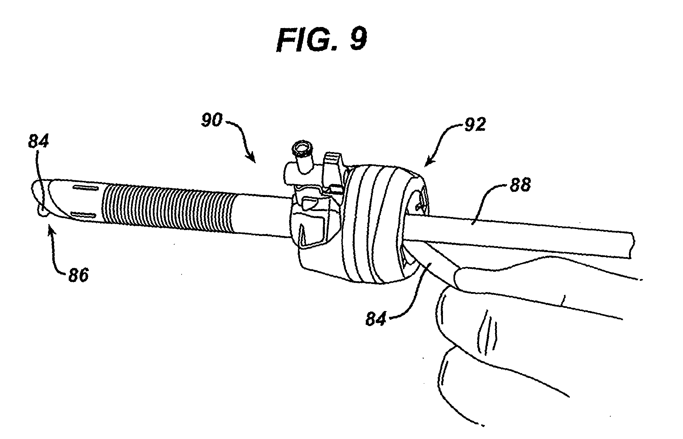

In use, as shown in FIG. 8, a sheath 84 (e.g., the sheath 50 of FIG. 5, the sheath 68 of FIG. 6, the sheaths 76 of FIG. 7, or any other sheath described herein) can be disposed around at least a distal end 86 of a surgical instrument 88. The sheath 84 can be disposed around the surgical instrument 88 such that, while the surgical instrument 88 is being introduced into a body cavity through an introducer device, e.g., a trocar 90, the sheath 84 can protect the surgical instrument's viewing element (at the instrument's distal end 86) from any fluid within the introducer device. The sheath 84 can also optionally capture and retain fluid within the introducer device using an absorbent outer layer as discussed above. After the surgical instrument's distal end 86 has reached a body cavity through the trocar 90, or at any other desired time before or after the surgical instrument's distal end 86 passes through the trocar 90, the sheath 84 can be torn away from the surgical instrument 88, such as shown in FIG. 9 where the sheath 84 is being proximally pulled from beyond a proximal end 92 of the trocar 90, thereby creating and/or restoring visual clarity through the surgical instrument's viewing element.

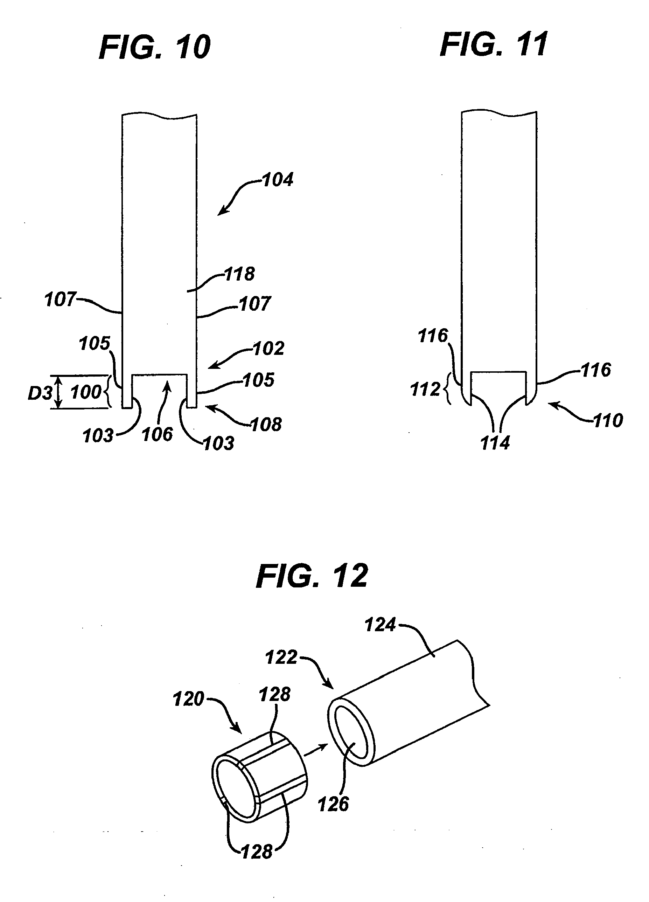

In another embodiment, illustrated in FIGS. 10-18, the protective element can be in the form of an extension element. Generally, as shown in FIG. 10, an extension element 100 coupled to a distal end 102 of a surgical instrument, e.g., an endoscope 104, can protect a viewing element 106 at the endoscope's distal end 102. The extension element 100 can be configured to shield the endoscope's viewing element 106 as the endoscope 104 passes through an introducer device to help prevent fluid within the introducer device from hindering the visual acuity of the viewing element 106. The extension element 100 can also be configured to be located outside the viewing element's viewing path, thereby providing protection to the viewing element 106 without obstructing visibility through the viewing element 100.

The extension element 100 can be formed from a variety of materials but is preferably formed from a biocompatible material. The extension element 100 can be made from any combination of rigid and/or flexible materials, but in an exemplary embodiment, the extension element 100 is formed from a rigid material to help open a trocar seal, as discussed further below, before the viewing element 106 at the endoscope's distal end 102 can contact the trocar seal and any fluid collected thereon. While the extension element 100 is preferably smooth, any portion (including all) of the extension element 100 can be textured and/or include depressions or protrusions to, for example, help grip a trocar seal or to help divert fluid away from the endoscope's viewing element 106.

The extension element 100 can have any size, shape, and configuration. Generally, the extension element 100 can be coupled to or formed on the endoscope's distal end 102 to form an extension that extends a distance D3 distally beyond the endoscope's distal end 102 and hence also beyond the viewing element 106. The extension distance D3 between the endoscope's distal end 102 and the extension element's distal end 108 can vary, but the distance D3 is preferably large enough to extend distally beyond the viewing element 106 at the endoscope's distal end 102. By way of example only, the distance D3 can be about 0.25 inches or about 6.67 x 10⁻⁶ inches (1/150,000 in.). The extension element 100 preferably extends circumferentially around the endoscope's distal end 102, e.g., as a cylindrical extension to match the typically cylindrical shape of the distal end 102 of the endoscope 104, to provide continuous protection around the viewing element 106. However, the extension element 100 can be in the form of one or more discrete extensions (e.g., rods or slats having any width) each extending beyond the endoscope's distal end 102 by the same or different distances. The extension element's inner and outer surfaces 103, 105 can be linear with the endoscope's inner surface (not shown) and outer surface 107, as shown in FIG. 10. Alternatively, as shown in FIG. 11, a portion of an extension element 112, such as an outer surface 116 at a distal end 110, can be non-linear, e.g., angled or curved inwardly toward an inner surface 114 of the extension element 112. An angled or curved outer surface can help divert fluid toward the extension element's outer surface 116 and away from a surgical instrument's viewing element.

As indicated above, the extension element 100 can be integrally formed with the endoscope 104, as illustrated for example in FIG. 10, in which case the extension element 100 can be made from the same material as a shaft 118 of the endoscope 104. Alternatively, as shown in FIG. 12, an extension element 120, similar to the extension element 100 of FIG. 10, can be fixedly or detachedly mated to a distal end 122 of a surgical instrument 124 to protect a viewing element 126 at the surgical instrument's distal end 122. The extension element 120 can include one or more channels or grooves 128 on its outside surface that can help divert fluid or other fluid encountered by the extension element 120 away from the viewing element 126.

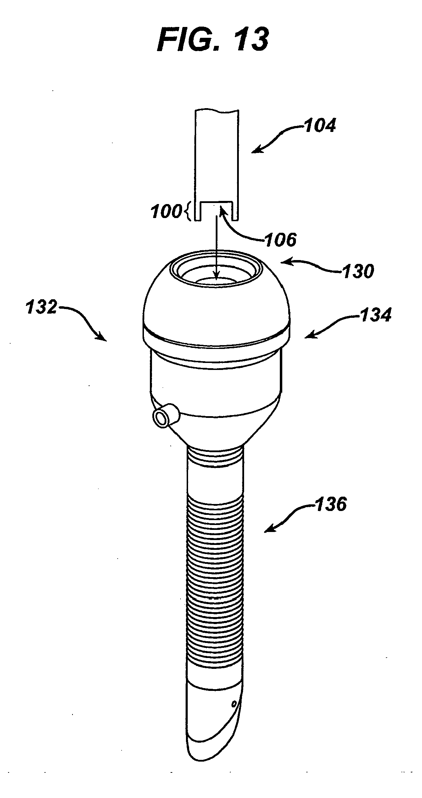

In use, as shown in FIG. 13, the extension element 100 (or any other extension element described herein) with the endoscope 104 (or any other surgical instrument as discussed above) mated thereto can be distally advanced in the direction of the directional arrow into a proximal end 130 of an introducer device, e.g., a trocar 132. As will be appreciated by a person skilled in the art, the trocar 132 can include any rigid and/or flexible cannulated introducer device configured to be inserted into a body cavity. Exemplary trocars are described in U.S. Application No. 11/855,777 entitled "Trocar Assembly" filed September 14, 2007, U.S. Application No. 11/952,464 entitled "Trocar Seal With Reduced Contact Area" filed December 7, 2007, U.S. Publication No. 2004/0230161 entitled "Trocar Seal Assembly" filed March 31, 2004, and U.S. Publication No. 2007/0185453 entitled "Conical Trocar Seal" filed on October 15, 2003, all of which are hereby incorporated by reference in their entireties.

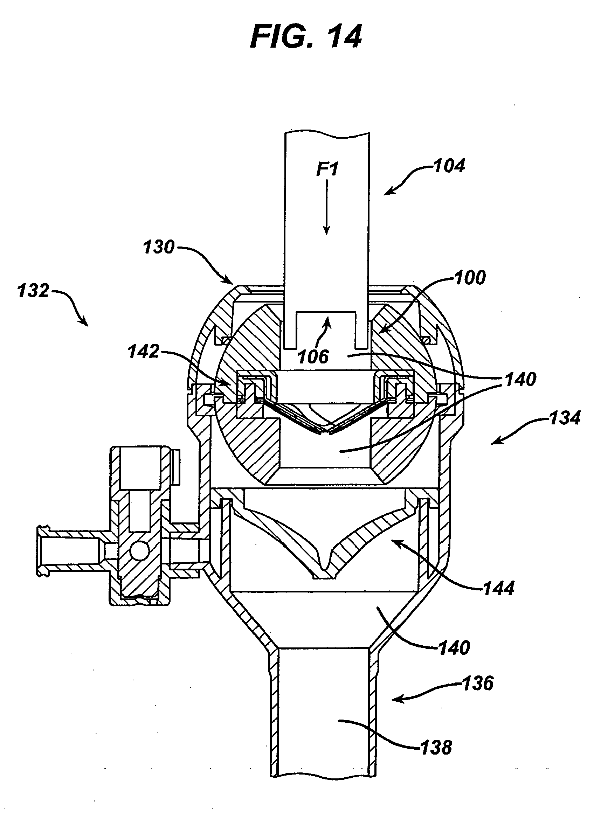

Generally, the trocar 132 can include a handle or trocar housing 134 with a cannula 136 that extends distally therefrom. As shown in FIG. 14, the cannula 136 can extend distally from the housing 134 to define an inner lumen or working channel 138 that is in communication with an inner lumen or working channel 140 defined by the housing 134. The working channels 138, 140 can be sized and configured to receive a surgical instrument, e.g., the endoscope 104. As discussed further below, the housing 134 can include proximal and distal seal assemblies 142, 144, at least partially positioned therein, that the extension element 100 can open as the extension element 100 is distally advanced through the housing's working channel 140 to help prevent fluid within the working channel 140 and/or collected on the proximal and distal seal assemblies 142, 144 from reducing visual clarity through the endoscope's viewing element 106. A person skilled in the art will appreciate that the trocar can include one or more seal assemblies of any type that can alone or in combination provide a zero-closure seal and an instrument seal, e.g., a duckbill seal, a flapper valve, a flapper door, a gel pad seal, an overlapping multi-layer seal, etc., that the extension element 100 can encounter prior to a surgical instrument's viewing element.

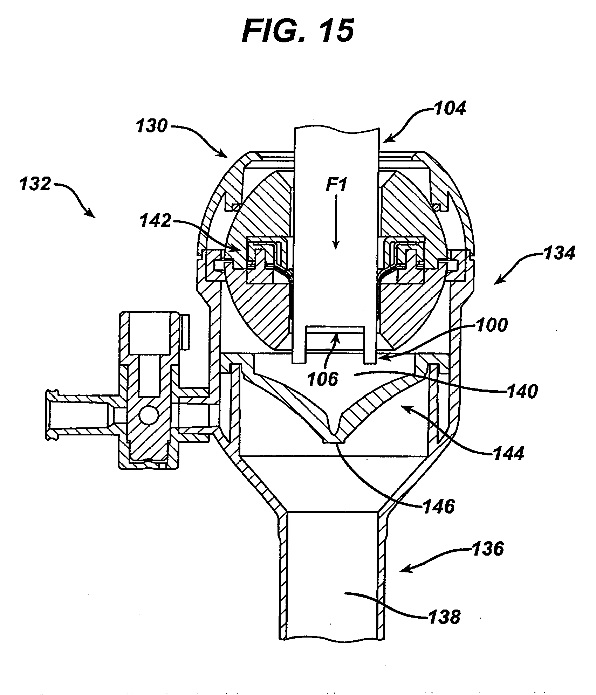

Generally, the proximal seal assembly 142 can be configured to cooperate with an exterior of any instrument inserted at least partially through the trocar 132 such that the proximal seal assembly 142 can sealingly engage the exterior of the instrument and thus can prevent the passage of fluid, including air, through the trocar housing 134 when the instrument is present within the trocar 132. Virtually any type of seal can be used to selectively seal the cannula's working channel 136. In one exemplary embodiment, the proximal seal assembly 142 can form a seal around an instrument inserted therethrough. FIGS. 14 and 15 illustrate one exemplary embodiment of such a seal assembly 142 having a conical shape. The seal can include a series of overlapping flexible elastomeric seal segments that are assembled in a woven arrangement to provide a complete seal body. The seal segments can be stacked on top of one another or woven together in an overlapping fashion to form a multi-layer conical shaped seal. The seal can also include a protective element, which can likewise be formed from multiple interwoven layers, positioned proximal of the seal for protecting the seal from accidental penetration or other damage as instruments are inserted therethrough.

As shown, for example in FIG. 14, the distal seal assembly 144 can be positioned distal to the proximal seal assembly 142, and it can be configured to allow for selective sealing of the working channel 140 of the trocar housing 134. Several types of seals can make up the distal seal assembly 144. In an exemplary embodiment, shown in FIGS. 14-18, the distal seal assembly 144 is a zero-closure seal, such as a duckbill seal. A person skilled in the art will appreciate that virtually any type of seal can form the distal seal assembly 144 including, but not limited to, duckbill seals, flapper valves, and flapper doors. Providing the trocar 132 with first and second seal elements can eliminate the need for the proximal seal assembly 142 to be a zero-closure seal. Thus, the proximal seal assembly 142 can only form a seal when an instrument is present in the trocar 132, and the distal seal assembly 144 can be adapted to seal the trocar 132 when no instrument is present.

A person skilled in the art will recognize that, while in an exemplary embodiment two seal assemblies 142, 144 are provided in the trocar 132, in other embodiments one seal assembly or more than two seal assemblies can also be used in the trocar 132. For example, as indicated above, a single seal can both function to seal the working channel of the trocar when no instruments are inserted therethrough and to also form a seal around an instrument inserted therethrough. One exemplary embodiment of such a seal is a gel pad seal as is known in the art.

In an initial position, illustrated in FIG. 14, an object, such as the endoscope 104, is not in contact with any portion of the proximal seal assembly 142. Turning to FIG. 15, a force generally in a direction F1 (e.g., a distal direction) can be applied to the endoscope 104, thus causing the endoscope 104 to move through the housing's proximal end 130 into the housing's working channel 140 toward the proximal seal assembly 142 in the general direction F1. The extension element 100 coupled to the endoscope 104 will come into contact with at least a portion of the proximal seal assembly 142, e.g., the seal segments, thus preventing the viewing element 106 from contacting the seal. The extension element 100, followed by at least a portion of the endoscope 104, will push the proximal seal assembly 142 open as it is advanced through the proximal seal assembly 142. In this way, the extension element 100 can contact the proximal seal assembly 142 before the distal end 102 of the endoscope 104 and can push open the proximal seal assembly 142 to help prevent the viewing element 106 from contacting the proximal seal assembly 142 and any fluid collected thereon.

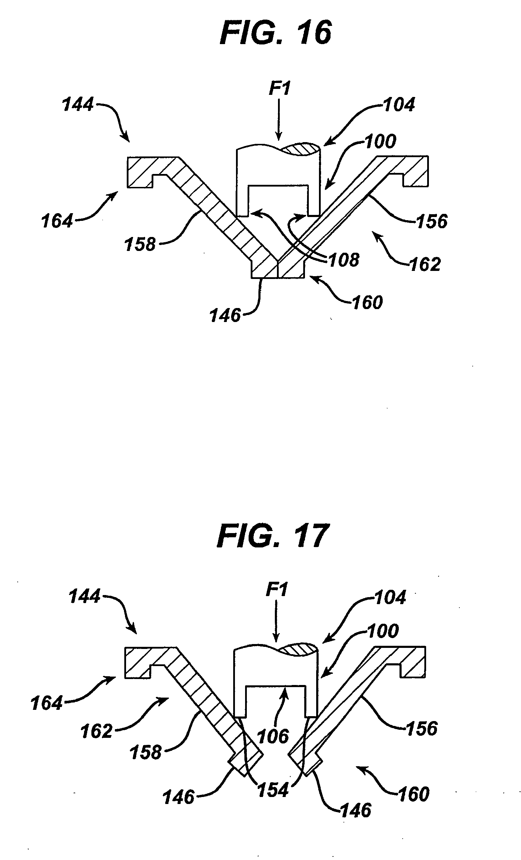

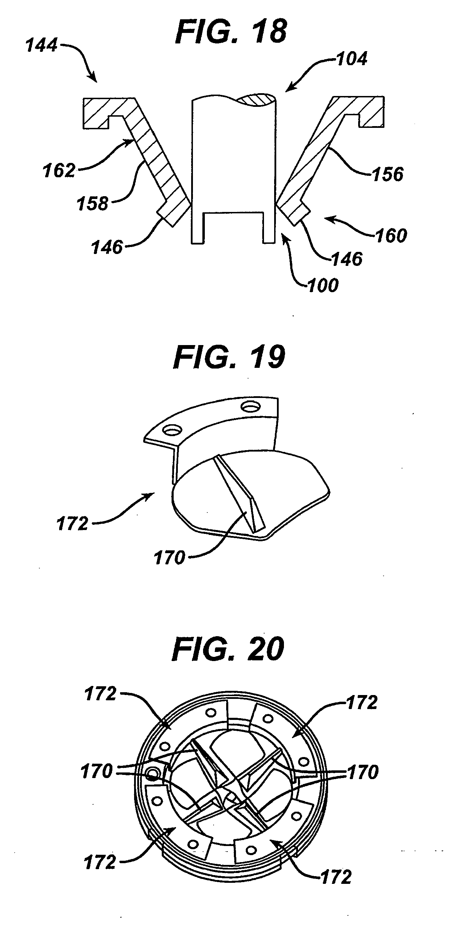

In an initial position, illustrated in FIG. 15, a seal face 146 of the distal seal assembly 144 is substantially closed and an object, such as the extension element 100 or the endoscope 104, is not in contact with any portion of the distal seal assembly 144. Turning to FIG. 16, a force generally in the direction F1 can be applied to the endoscope 104, thus causing the extension element 100 and the endoscope 104 to move toward the seal face 146 in the general direction F1 until the distal end 108 of the extension element 100 is in contact with at least a portion of the distal seal assembly 144. As the force in the direction F1 continues to be applied to the endoscope 104, the extension element 100 continues to move toward the seal face 146, as illustrated in FIG. 17, and can result in the distal seal assembly 144 being in an opening position in which the seal face 146 is open, the distal seal assembly's seal elements 156, 158 move further apart than they were in the initial position, and the extension element 100 moves distally along the seal. In this way, the extension element 100 can prevent the endoscope's viewing element 106 from coming into contact with the distal seal assembly 144 and any fluid collected thereon. Once the extension element 100 and/or the endoscope is moved to its desired location beyond the seal face 146, the distal seal assembly 144 is in a final position, illustrated in FIG. 18. In the final position the seal face 146 can remain open and the seal elements 156, 158 can continue to be further apart than they were in the initial position.

Once the endoscope 104 is done being used, it can be removed from the seal assemblies 142, 144 in much the same manner as it was introduced to the seal assemblies 142, 144.

Whether in the initial position, the contacting position, the opening position, or the final position, in an exemplary embodiment a system including the proximal and distal seal assemblies 142, 144 and the endoscope 104 generally creates a closed cavity between an environment below a distal end 160 of the distal seal assembly's seal body 162 and an environment above a proximal end of the proximal seal 142. For example, as shown in FIGS. 15 and 16, in both the initial and contacting positions the closed cavity is formed by the seal face 146 of the distal seal assembly 144 being substantially closed. However, in the open and final positions as seen in FIGS. 17 and 18, the closed cavity will be formed by the proximal seal assembly 142.

In another embodiment, illustrated in FIGS. 19 and 20, the protective element can be disposed on a trocar seal assembly. For example, an extension or camming rib 170 can be formed on a surface of a seal assembly to prevent fluid in the seal opening from being transferred to a distal face of an instrument inserted therethrough. In the illustrated embodiment, the seal assembly includes a multi-layer seal and a multi-layer protective element, as discussed above regarding FIGS. 14 and 15. One or more camming ribs 170 can be formed on a proximal-facing surface of a protective element 172. The camming rib 170 can allow the protective element 172 to be opened prior to the instrument contacting the opening in the seal so that fluid extending across the opening is prevented from contacting the distal face of the surgical instrument as it is passed through the opening. This can be achieved because the camming ribs 170, when contacted by the instrument, will cause the seal to open more rapidly, thus allowing fluid in the opening to break or otherwise move away from the opening. The camming ribs also provide point contact, rather than surface contact, with the instrument, thus reducing the likelihood of fluid on the surface from being transferred to the instrument. In one embodiment, the camming ribs 170 are of the same size and shape. In another embodiment, illustrated in FIG. 20, the camming ribs 170 can have a different size and shape such that the protector is opened asymmetrically, thereby making it more difficult for fluid to remain within the opening of the sealing element. The camming ribs 170 can also be located at various locations, but as shown are equally spaced circumferentially around the proximal surface of the protective element 172.

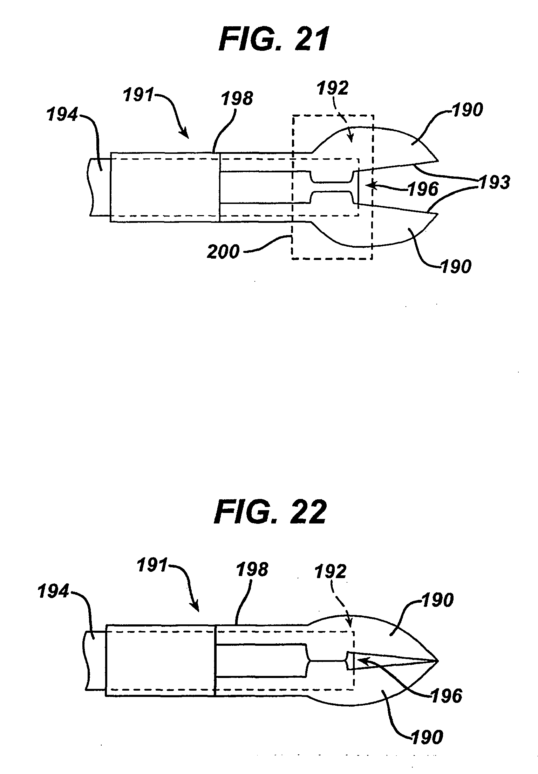

In another embodiment, illustrated in FIGS. 21-31, the protective element can be in the form of movable jaws. Generally, as shown in FIGS. 21 and 22, movable jaws 190 coupled to a distal end 192 of a surgical instrument, e.g., an endoscope 194, can protect a viewing element 196 at the endoscope's distal end 192. In an exemplary embodiment the jaws 190 can be configured to move between an open position (FIG. 21), in which the endoscope's distal end 192 can be exposed to allow viewing through the viewing element 196, and a closed position (FIG. 22), in which the jaws 190 can enclose the endoscope's distal end 192. In other words, the jaws 190 in the closed position can shield the endoscope's viewing element 196 from an external environment (e.g., provide a fluid seal around the endoscope's distal end 192), such as when the endoscope 194 passes through an introducer device, to help protect the viewing element 196 from becoming obscured, damaged, or otherwise adversely affected by fluid while the endoscope 194 passes into or out of a body cavity. In the open position, the jaws 190 can allow the viewing element 196 to be exposed to an external environment outside an introducer device.

The jaws 190 can be formed from any type and any combination of preferably biocompatible materials. The jaws 190 can be rigid or flexible, but the jaws 190 are preferably rigid to help prevent the jaws 190 from unintentionally bending or otherwise unintentionally moving between the closed and open positions to help avoid unintentionally obstructing the viewing element's viewing path, releasing a fluid seal provided by the jaws 190 in the closed position, and/or producing any other undesirable effects.

Although two jaws 190 are shown coupled to the endoscope 194 in FIGS. 21 and 22, any number of jaws 190 can be coupled to the endoscope 194. The jaws 190 can also have any size. The jaws 190 preferably have a size to allow for enclosure of the endoscope's distal end 192 when the jaws 190 are in the closed position and to be positioned outside the viewing element's viewing path when the jaws 190 are in the open position. The jaws 190 can also have any shape. The size and/or shape of one of the jaws 190 can be the same or different from any of the other jaws 190 so long as the jaws 190 can provide a protective cover over the endoscope's viewing element 196 when the jaws 190 are in the closed position. As shown in FIGS. 21 and 22, the jaws 190 have a substantially hollow body 191 configured to receive the endoscope 194 and concave inner surfaces 193 that when together in the closed position can form a tapered, substantially tubular oblong member.

The jaws 190 can be coupled to the endoscope 194 in any way, such as via an end cap or housing 198 coupled to the endoscope 194. The jaws 190 can be integrally formed with the housing 198, as illustrated in FIGS. 21 and 22, or the jaws 190 can be fixedly or detachedly coupled to the housing 198. The housing 198 can be formed from any type and any combination of preferably biocompatible materials. The housing 198 can be rigid or flexible, but the housing 198 is preferably rigid to help stabilize the jaws 190 in the jaws' closed and open positions. The housing 198 can also have any size and shape, but the housing 198 is preferably cylindrical to match the typically cylindrical shape of the distal end 192 of the endoscope 194. The housing 198 can extend around any portion of the endoscope's longitudinal length.

As mentioned above, the housing 198 can be detachably or fixedly coupled to the endoscope 194, preferably at the endoscope's distal end 192. If the housing 198 is detachably coupled to the endoscope 194, the housing 198 can be detached from the endoscope 194 and optionally replaced with another, similar housing. Such replacement can be advantageous, for example, if the housing 198 and/or the jaws 190 get damaged or otherwise become unusable or undesirable. Detachably coupling the housing 198 to the endoscope 194 can also allow the housing 198 to be retrofitted to existing surgical instruments and/or to be detached from the endoscope 194 and be coupled to one or more other surgical instruments (preferably after sterilizing the housing 198).

Whether integrally formed with the housing 198, otherwise coupled to the housing 198, or coupled to the endoscope 194 without the housing 198 (e.g., as integrally formed with the endoscope 194 at its distal end 192), the jaws 190 can be biased to the open position where the jaws 190 are outside the viewing element's viewing path, e.g., are invisible to the viewing element 196. In this way, when a force is not being applied to the jaws 190, the jaws 190 can be in the open position so as to not obstruct the viewing element's viewing path.

The jaws 190 can move between the open and closed positions in a variety of ways. For example, the jaws 190 can be manually movable between the open and closed positions by pinching, squeezing, or otherwise moving the jaws 190 with hands or a surgical tool. In an exemplary embodiment, the jaws 190 can be coupled to a sheath or outer sleeve similar to the outer sleeve 20 discussed above. The outer sleeve can allow manual movement of the jaws 190 between the open and closed positions from a location proximal to the jaws 190. Such an outer sleeve can couple to the housing 198 at the housing's proximal end and extend around the endoscope 194 to the endoscope's proximal end and/or to a location outside an introducer device used to introduce the endoscope 194 into a body cavity such that the outer sleeve can be manipulated from outside the body cavity.

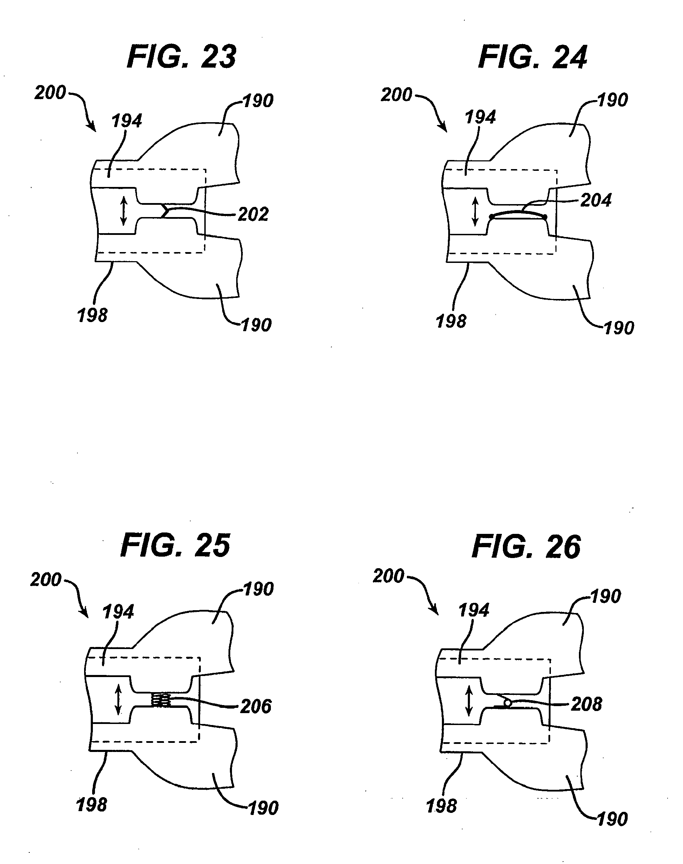

In an exemplary embodiment, a compliant mechanism coupled to the jaws 190 and disposed within the housing 198 can move the jaws 190 relative to each other. FIGS. 23-26 illustrate various exemplary embodiments of compliant mechanisms. The compliant mechanism can include a single material (e.g., a living hinge as shown in FIG. 23), or it can be any type of flexible member extending between the jaws 190 (e.g., a spring as shown in FIGS. 24-26). The compliant mechanisms shown in FIGS. 23-26 are each located substantially at a distal end 204 of the jaws 190, but a person skilled in the art will appreciate that a compliant mechanism can be coupled to the jaws 190 at any location as appropriate to allow movement of the jaws 190. As shown in FIG. 23, a hinge mechanism such as a flexure bearing or a living hinge 202 can be used to move the jaws 190 between the open and closed positions. The living hinge 202 can have any shape and size and can be formed from any type and any combination of materials, preferably a flexible, biocompatible material, e.g., a polymer. FIGS. 24-26 illustrate the compliant mechanism as various springs: a leaf spring 204 in FIG. 24, a compression spring 206 in FIG. 25, and a torsion spring 208 in FIG. 26. The springs 204, 206, 208 can each have any shape that allows the jaws 190 to move between the open and closed positions. For example, the spring can be in the form of a coil or helical spring having a cylindrical shape, although the spring can have other shapes, such as conical or dual conical, and have individual coils of any shape, such as elliptical or rectangular. Other examples of springs include an elastic band/thread/cord, a bellows, a volute spring, and other similar types of flexible elastic objects.

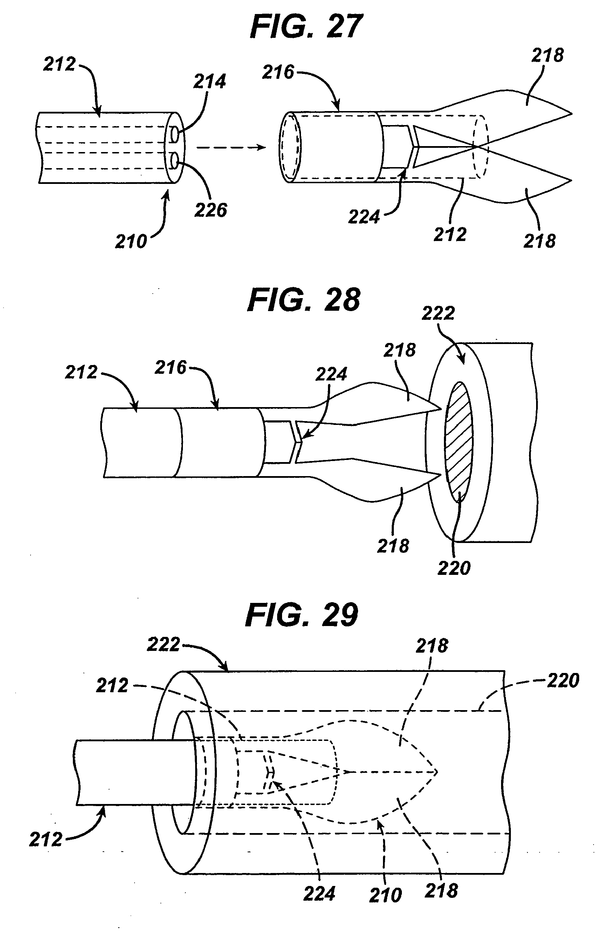

In use, as shown in FIG. 27, at least a distal end 210 of a surgical instrument 212 having a viewing element 214 at its distal end 210 can be disposed in an end cap 216 having a pair of movable jaws 218 formed therein or coupled thereto. The jaws 218 are shown in a closed position in FIG. 27, but the jaws 218 can be in an open or a closed position when the surgical instrument 212 is disposed within or otherwise coupled to the end cap 216. As shown in FIG. 28, the jaws 218 can be distally advanced into a pathway 220 extending through an introducer device, e.g., a trocar 222, along with the surgical instrument 212. Because the jaws 218 are biased to an open position, a compliant mechanism, e.g., a living hinge 224, coupling the jaws 218 is also in an open, uncollapsed position outside the trocar's pathway 220. When one or more forces act upon the jaws 218, such as when the jaws 218 are at least partially introduced into the trocar's pathway 220, the hinge 224 can begin to collapse, and the jaws 218 can begin to move from the open position to the closed position. As shown in FIG. 29, when the jaws 218 have been completely disposed within the trocar's pathway 220, the jaws 218 can be in the closed position, thereby providing a fluid seal around the surgical instrument's distal end 210.

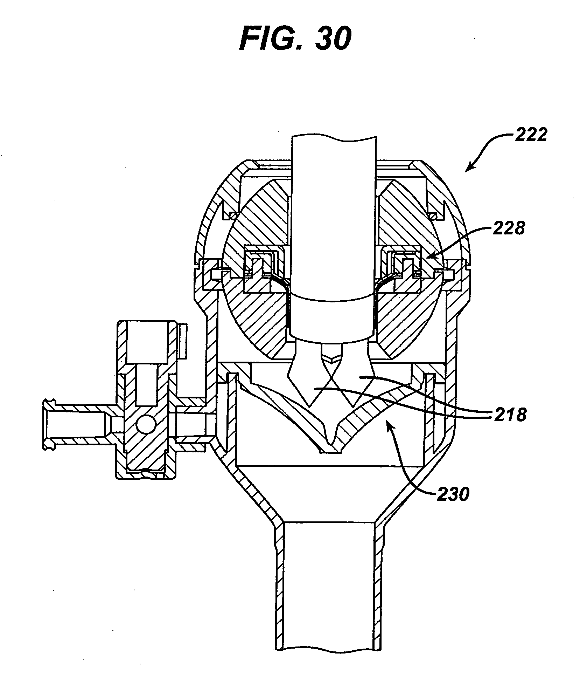

Similar to the trocar 132 discussed above, the trocar 222 can include one or more seal assemblies disposed therein. Because the jaws 218 extend distally beyond the surgical instrument's distal end 210, the jaws 218 can open one or more seals within the trocar 222 and help prevent any fluid deposited on the seal(s) from collecting on or otherwise interfering with the surgical instrument's viewing element 214 and with any other elements at the surgical instrument's distal end 210, such as a working channel 226 extending longitudinally through the surgical instrument. As shown in FIG. 30, distal advancement of the surgical instrument 212 into the trocar 222 has allowed the jaws 218, shown in a substantially closed position extending around the surgical instrument's distal end, to open a proximal seal assembly 228 similar to the extension element 100 opening the proximal seal assembly 142 as discussed above. Continued distal advancement of the surgical instrument 212 can allow the jaws 218 to open a distal seal assembly 230 similar to the extension element opening the distal seal assembly 244 as discussed above. Also similar to the trocar 132 discussed above, the trocar 222 can include any number and any type of seal assemblies.

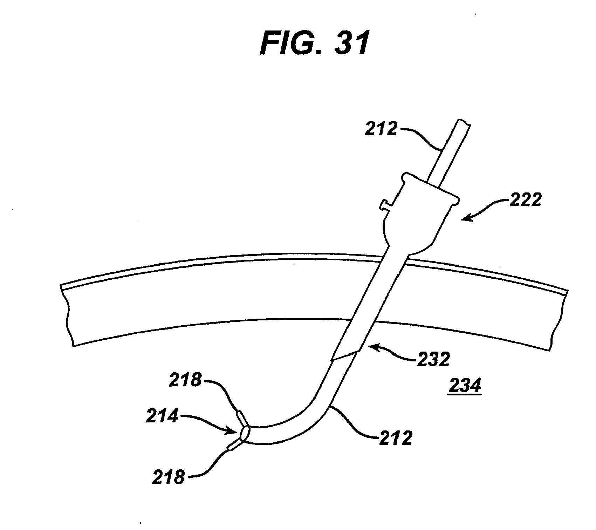

As shown in FIG. 31, when the jaws 218 move distally out of the pathway 220 beyond a distal end 232 of the trocar 222 inside a body cavity 234 (or elsewhere), the jaws 218 can move from the closed position to the open position, thereby providing an unobstructed viewing path for the surgical instrument's viewing element 214 within the body cavity 234. With the jaws 218 in the open position, other surgical instruments can be disposed through the surgical instrument's working channel 226 into the body cavity 234.

The surgical instrument 212 can be removed from the body cavity 234 through the trocar 22, with the jaws 218 in a closed position within the trocar 222, in much the same manner as the surgical instrument 212 was introduced through the trocar 222.

The device disclosed herein can also be designed to be disposed of after a single use, or it can be designed to be used multiple times. In either case, however, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A surgical device, comprising:
a surgical instrument configured to pass through a working channel of an introducer device extending into a body cavity; and
a protective element coupled to the surgical instrument and configured to prevent fluid from contacting a distal end of the surgical instrument while the surgical instrument is being passed through the introducer device.

2. The device of claim 1, wherein the surgical instrument comprises a scope with a viewing element at a distal end of the scope.

3. The device of claim 1, wherein the protective element is configured to prevent fluid from contacting the distal end of the surgical instrument while the distal end of the surgical instrument is disposed in the body cavity.

4. The device of claim 1, wherein the protective element is disposed on the distal end of the surgical instrument, wherein the protective element comprises a plurality of movable protective members.

5. The device of claim 4, wherein the protective members comprise a plurality of lenses stacked on top of one another.

6. The device of claim 5, wherein each lens is hingedly coupled to a support mated to the distal end of the surgical instrument.

7. The device of claim 1, wherein the protective element is configured to be removed from the surgical instrument.

8. The device of claim 1, wherein the protective element comprises an absorbent material disposed on an outside surface of the protective element for absorbing fluid.

9. The device of claim 1, wherein the protective element comprises a tear-away sheath disposed over at least a distal portion of the surgical instrument.

10. The device of claim 1, wherein the protective element comprises movable jaws coupled to the distal end of the surgical instrument and configured to move between a closed position in which the jaws enclose the distal end of the surgical instrument and an open position in which the distal end of the surgical instrument is exposed.

11. A surgical device, comprising:
a scoping device having a viewing element at a distal end; and
at least two optically clear protective elements coupled to the distal end of the scoping device and configured to provide a barrier to protect the viewing element from fluid in an external environment, the optically clear protective elements being sequentially movable.

12. A surgical device, comprising:
a scoping device having a viewing element at a distal end, the scoping device being configured to be disposed in a body through a working channel of an introducer device; and
a protective element coupled to the scoping device and movable between a first configuration in which the protective element provides a fluid seal around the viewing element while the distal end of the scoping device is disposed through a working channel of an introducer device, and a second configuration in which the viewing element is exposed to a fluid environment surrounding the distal end of the scoping device.

13. A surgical device, comprising:
a scoping device having a viewing element at a distal end, the scoping device being configured to be disposed in a body through a working channel of an introducer device; and
a diverting mechanism located on the distal end of the scoping device and effective to open a seal in an introducer device to prevent the viewing element from contacting the seal.

14. A surgical method, comprising:
passing a surgical instrument through an introducer device having a working channel extending into a body cavity, wherein a distal end of the surgical instrument includes a protective element that prevents fluid from contacting the distal end of the surgical instrument while the surgical instrument is being passed through the introducer device.

15. A surgical device, comprising:
a trocar having a working channel configured to extend into a body cavity and to allow passage of a surgical instrument therethrough; and
a seal assembly disposed in the trocar having a seal with an opening configured to form a seal around a surgical instrument passed through the trocar, and a protective element including at least one camming rib formed thereon and configured to contact the surgical instrument prior to the surgical instrument contacting the seal.
